# EUROPEAN PATENT SPECIFICATION

(11) **EP 2 697 382 B1**
(45) Date of publication and mention of the grant of the patent: **07.03.2018**
(21) Application number: 12771384.0
(22) Date of filing: 22.03.2012
(51) Int. Cl.: A61K 38/28, A61M 5/145, A61M 5/172

(54) **PRE-OPERATIVE USE OF METABOLIC ACTIVATION THERAPY**
PRÄOPERATIVE ANWENDUNG EINER STOFFWECHSELAKTIVIERUNGSTHERAPIE
UTILISATION PRÉOPÉRATOIRE DE THÉRAPIE D'ACTIVATION MÉTABOLIQUE

(30) Priority: 12.04.2011 US 201161516955 P
(43) Date of publication of application: 19.02.2014
(73) Proprietor: Aoki, Thomas, T., Sacramento, CA 95864 (US)
(72) Inventor: Aoki, Thomas, T., Sacramento, CA 95864 (US)
(74) Representative: HGF Limited
(86) International application number: PCT/US2012/000156
(87) International publication number: WO 2012/141764

(56) References cited:
- WO-A2-03/084459
- US-A1- 2006 122 099
- US-B2- 6 579 531
- CAN M F ET AL: "Preoperative administration of oral carbohydrate-rich solutions: Comparison of glucometabolic responses and tolerability between patients with and without insulin resistance", NUTRITION, ELSEVIER INC, US, vol. 25, no. 1, 1 January 2009 (2009-01-01), pages 72-77, XP025742246, ISSN: 0899-9007, DOI: 10.1016/J.NUT.2008.07.021 [retrieved on 2008-10-11]
- RAJU ET AL.: 'Perioperative blood glucose monitoring in the general surgical population.' JOURNAL OF DIABETES SCIENCE AND TECHNOLOGY vol. 3, no. 6, November 2009, pages 1282 - 1287, XP055138119

## Description

### BACKGROUND OF THE INVENTION

Mortality rates in diabetic patients have been estimated to be up to 5 times greater than in nondiabetic patients, often related to the end-organ damage caused by the disease. Chronic complications resulting in microangiopathy (retinopathy, nephropathy, and neuropathy) and macroangiopathy (myocardial infarction, strokes, peripheral vascular disease) directly increase the need for surgical intervention and concomitantly the occurrence of surgical complications due to infections. Infections account for 66% of postoperative complications and nearly one quarter of perioperative deaths in diabetic patients. Data suggest impaired leukocyte function, including altered chemotaxis and phagocytic activity. Tight control of circulating glucose has been the mainstay of interventions to minimize infection.

It is incorrectly assumed that high glucose levels (hyperglycemia) occur only in individuals with diabetes. Indeed, there is a large group of patients who are not or are not known to be diabetic who will undergo surgery and other insulin resistance inducing procedures including those requiring hospitalization with circulating glucose levels as high as or higher than 150 mg/dl. These patients would typically be older than 50 years of age and may be experiencing increased insulin resistance for any of a number of reasons other than diabetes including but not limited to, the taking of insulin resistance inducing medications such as prednisone or hydrocortisone; experiencing significant pain which causes insulin insensitivity; concurrently suffering one of many kinds of infectious diseases; experiencing one of many kinds of trauma which can trigger high circulating glucose levels; aging normally without any diagnosed diabetes (insulin resistance of aging); experiencing obesity, lack of exercise and/or stress; patients diagnosed with MELAS syndrome and endocrine dysfunction; patients suffering from one of many forms of pancreatitis resulting in impaired insulin production.

Diabetic individuals are subject to longer hospitalizations and major operations more frequently than those without diabetes. Current guidelines recommend that glucose control (140-180 mg/dl) be maintained, primarily by the use of insulin, throughout the hospital stay to assure a full recovery and early release from hospitalization. Insulin may be administered in multiple subcutaneous injections or by continuous intravenous infusion (IV). With the increased use and accuracy of bedside glucose monitoring, IV insulin therapy has become the preferred method of insulin delivery. The Endocrine Society's recommendation is based on several observational and prospective clinical trials that have shown that plasma glucose levels above 180 mg/dl are associated with increased risk of infections, longer hospital stays and increased mortality.

IV insulin therapy requires the use of electro-mechanical pumps that deliver the prescribed volumes accurately. Regular insulin is administered by continuous infusion, with changes in the rate based on the bedside glucose results. At the same time, the patient is also given IV glucose, in order to prevent hypoglycemia and provide fluids. IV insulin therapy is preferred because it is more convenient, safer, and eliminates complex subcutaneous insulin injection orders.

Studies have shown that diabetes mellitus is an independent predictor of postoperative myocardial ischemia among patients undergoing cardiac and non-cardiac surgery, in addition to postoperative infections after cardiac surgery. Tighter postoperative glycemic control has been shown to have a significant effect on reducing the incidence of many of these complications in a variety of surgical populations.

A meta-analysis of 15 studies reported that hyperglycemia increased both in-hospital mortality and incidence of heart failure in patients admitted for acute myocardial infarction, independent of a previous diagnosis of diabetes mellitus (DM), demonstrating that the diagnosis of diabetes mellitus is not as important as controlling circulating glucose concentrations.

Although the benefits of tighter glycemic control have been well documented in many patient populations, the optimal range for glucose has yet to be well defined. A study led by Greet Van den Berghe, MD, PhD, conducted in Leuven, Belgium found that when adult surgical ICU patients' glucose levels were maintained between 80 and 110 mg/dL by means of an insulin infusion, it reduced ICU mortality by 42 percent and in-hospital mortality by 34 percent (N Engl J Med. 2001;345:1359-1367). Other studies showed similar results with different levels of glycemic control. More sobering was a large multinational study called the Normoglycemia in Intensive Care Evaluation-Survival Using Glucose Algorithm Regulation (NICE-SUGAR) trial (N Engl J Med. 2009; 360:1283-1297). This study tested the hypothesis that intensive glucose control reduces mortality at 90 days. But unexpectedly, it showed that people with intensive glycemic control had more frequent severe hypoglycemia as well as higher mortality. Balancing the risks of hypoglycemia against the known benefits in morbidity and mortality is the goal, and, although intensive glycemic control continues to be standard of care, current consensus guidelines recommend less stringent glycemic goals, i.e. 140-180 mg/dl.

Even nondiabetic patients, because of the reasons enumerated above, may become hyperglycemic perioperatively and during other stress inducing procedures including those requiring hospitalization. Multiple randomized controlled studies have shown that controlling glucose levels in all patients, not merely those with diabetes mellitus (DM), impacts the outcome of surgical patients and patients undergoing other stress inducing procedures including those requiring hospitalization who are critically ill. This effect does not appear to be related to the dose of insulin but rather to the absolute level of plasma glucose achieved. Yet frequently, optimal control cannot be obtained because of a fear of hypoglycemic reactions.

Anesthetic agents can affect glucose metabolism through the modulation of sympathetic tone. In vitro data suggest that inhalational agents suppress insulin secretion. The resulting relative insulin deficiency often leads to hyperglycemia. This deficiency is compounded in diabetic patients, particularly those with insulin resistance, raising the risk of ketoacidosis. The use of regional anesthesia or peripheral nerve blocks may mitigate these concerns, but there is no data that suggests that these forms of anesthesia improve postoperative survival in patients with diabetes mellitus.

Given that diabetic patients present preoperatively with a variety of diabetes treatment regimens and are scheduled for surgery, angioplasty, stent placement, and other stress inducing procedures requiring hospitalization at varying times of the day, there is no established consensus for optimal management during the preoperative phase of an operation. Aside from diabetic patients, there are a large number of non-diabetic patients who are treated during and after the operation with IV insulin but who are also predisposed to hypoglycemia or excessive hyperglycemia with similarly increased mortality. Currently general management principles are used to minimize the likelihood of hypoglycemia and to limit the incidence of excessive hyperglycemia. But decisions about infusing insulin and glucose during and after operations are heavily influenced by concerns of overmedication and the threat of hypoglycemia and the associated increased mortality. Although the majority of high risk patients for hyperglycemia or hypoglycemia have diabetes mellitus, there is a large population of non-diabetic patients who are at risk as well.

What is needed is an insulin treatment regimen which can be used to biochemically prepare diabetic and non-diabetic patients for surgery and other stress inducing procedures including those requiring hospitalization, while minimizing both hyperglycemia and hypoglycemia during and after the surgery or other stress inducing procedures including those requiring hospitalization. Ideally such an insulin treatment regimen would allow for restoration toward normality of the biochemistry and physiology of these individuals resulting in the control of plasma glucose to levels lower than can currently be obtained with minimal threat of hypoglycemia. The stress on nursing personnel to make up to 48 or more accurate plasma glucose measurements a day, calculate appropriate insulin doses and glucose amounts to be administered, and then administer both could result in a considerable number of mistakes and subsequent harm to the patient. The preoperative insulin treatment regimen would be more valuable if it prepared the patients biochemically/physiologically for a given surgical procedure or other stress inducing procedures including those requiring hospitalization thereby obviating the necessity for achieving "tight" glucose control during and after surgery and other stress inducing procedures including those requiring hospitalization. As a result of such preemptive treatment, there should be fewer nursing errors and less potentially damaging outcomes to the patients. Finally such an insulin dosage regimen would lower overall medical costs by decreasing hospital length of stay as biochemically more normal individuals should heal faster and have fewer postoperative complications.

### SUMMARY OF THE INVENTION

Insulin is used in a method of treatment for treating subjects who are scheduled for surgery or some other physiologically stressful procedures including those requiring hospitalization wherein the subject is experiencing elevated circulating glucose levels. The method includes having the subject ingest a carbohydrate containing meal capable of increasing circulating glucose levels in the subject. The carbohydrate containing meal contains between 20 and 100 grams of glucose. The method further includes periodic monitoring of those circulating glucose levels. A series of intravenous pulses of insulin are given to the subject by an insulin infusion device. The method includes selecting the amount of insulin in each pulse, the interval between pulses and the amount of time to deliver each pulse to the subject so that a monitoring device which measures the therapeutic efficacy of the method provides physiological evidence that the treatment is effective. The subject may suffer from diabetes.

One such monitoring device of therapeutic efficacy measures the subject's circulating glucose level which initially rises and then falls by an amount equal to 50 mg/dl or more within two hours of administering the first pulse of the series of pulses. Another such monitoring device of therapeutic efficacy indicates that the subject's respiratory quotient rises to a level higher than 0.88.

The subject is given the series of insulin pulses between 2 and 8 times on at least one treatment day prior to surgery or other stress inducing procedure including those requiring hospitalization.

The insulin infusion device includes any of a number of devices such as a syringe pump to deliver the pulses of insulin. The device used to dispense insulin can comprise a programmable processor unit, and the amount of time in each pulse, the time interval between pulses and the amount of time to deliver each pulse are controlled by the programmable processor unit.

The insulin in each pulse is between 1 and 200 milliunits per kilogram of body weight of the subject, the series of pulses are delivered over a period of 6 to 180 minutes, and each pulse of the series of pulses is delivered to the patient every 3 to 30 minutes.

The information can be transferred automatically between a circulating glucose meter and the infusion device of the current invention.

Insulin is used in a method of treatment of subjects suffering from diabetes which subjects are scheduled for surgery or other stress inducing procedures including those who require hospitalization wherein the method includes subjects who have been treated with Metabolic Activation Therapy over an extended period of time for the purpose of treating complications of diabetes, including but not limited to at least one of the following such as improving energy levels of the subject; improving one or more of confusion, weakness, disorientation, or cognitive function in said subject suffering dementia; correcting over utilization of fatty acids associated with heart disease and cardiovascular disease; slowing the progression of nephropathy thereby reducing the risk of end stage renal disease; stabilizing or reversing the progression of retinopathy; improving the symptoms of autonomic neuropathy; improving or arresting the progression of peripheral neuropathy; and treating wounds promoting healing and avoiding amputations. Metabolic Activation Therapy comprises the steps of the ingestion by the subject of a meal capable of increasing circulating glucose levels in the subject combined with periodic monitoring of circulating glucose levels in said subject and delivery of the insulin to the subject in a series of intravenous pulses of insulin by an infusion device wherein the amount of said insulin in each pulse, the interval between pulses and the amount of time to deliver each pulse to the subject are selected so that a monitoring device to measure therapeutic efficacy provides physiological evidence that the treatment is effective.

The said monitoring device to measure therapeutic efficacy indicates that the subject's circulating glucose level initially rises and then falls by an amount equal to 50 mg/dl or more or it indicates that the subject's respiratory quotient (RQ) rises to a level higher than 0.88.

The extended period of time for said patient is more than 3 weeks duration, the amount of insulin used in each pulse for Metabolic Activation Therapy is between 1 and 200 milliunits per kilogram of body weight, the infusion device comprises a programmable processor unit and the amount of insulin in each pulse, the time interval between pulses and the amount of time to deliver each pulse to the subject are controlled by said programmable processor unit.

One series of pulses of insulin for Metabolic Activation Therapy is delivered over a period of 6 to 180 minutes and each pulse of said series of pulses is delivered every 3 to 30 minutes. The amount of ingested glucose is between 20 and 100 grams of glucose.

Metabolic Activation Therapy can be of a form wherein information is transferred automatically between a circulating glucose meter and said infusion device.

### DESCRIPTION OF THE DRAWINGS

Herein follows a brief description of the drawings:
**FIG. 1** is a schematic block diagram of a programmable insulin pump utilizing a syringe plunger-type of mechanism and programmed to deliver insulin according to the present invention.
**FIG. 2** is a graphical representation of the incidence of hypoglycemia in 20 patients before and after receiving Metabolic Activation Therapy
**FIG. 3** is a graphical representation of serial changes in postoperative mean plasma glucose in the treatment group compared to the control group.
**FIG. 4** is a graphical representation of the mean plasma glucose level of patients on the first day, the first two days and the entire seven days after the operation.
**FIG. 5** is a graphical representation of the I/G (insulin/glucose) and I/C (insulin/carbohydrate) ratios of patients on the first day, the first two days, and the entire seven days after the operation.
**FIG. 6** is a graphical representation of the targeted perioperative levels of plasma glucose compared to the definition of hypoglycemia from seven different studies.
**FIG. 7** is a graphical representation that demonstrates the number of times plasma glucose was measured perioperatively in seven different studies.

### DETAILED DESCRIPTION OF THE PREFERRED EMBODIMENTS

In the preferred embodiment of the current invention Metabolic Activation Therapy, consisting of an insulin infusion device is used to inject intravenous insulin into subjects including but not limited to 1) diabetic and 2) non-diabetic patients over the age of 50 years scheduled for an elective physiological stress inducing surgical/medical procedure and an RQ (Respiratory Quotient) instrument that provides evidence that the treatment is effective, in a clinic or hospital to prepare patients for surgery and other stress inducing medical procedures including those requiring hospitalization. Preferably, the patient will have at least two days of treatment within one to two weeks before a scheduled operation or other stress inducing medical procedures. At the attending physician's discretion, at times a single treatment day will suffice.

At the discretion of the attending physician, patients who are already receiving Metabolic Activation Therapy on a weekly or bi-weekly basis for an extended period of time, depending on patient needs, may not need to change their normal regimen. An extended period of time is any amount of time in excess of 3 weeks of treatment. Metabolic Activation Therapy is a well-known long term treatment of diabetic patients and in many cases has been used in excess of 10 and even 20 years in individual patients to effectively treat many of the debilitating complications of diabetes. US Patent 6,579,531 details a method for correcting over utilization of fatty acids associated heart disease and cardiovascular disease in both diabetic and non-diabetic patients using Metabolic Activation Therapy. US Patent 6,582,716 similarly details a method for treating wound, promoting healing and avoiding amputations in diabetic and non-diabetic patients using Metabolic Activation Therapy US Patents 6,613,342 and 6,821,527 detail a system and method for treating kidney disease or nephropathy in diabetic and non-diabetic patients which slows the progression of nephropathy reducing the risk of end stage renal disease, US Patents 6,613,736 and 6,967,191 describe a method for treating eye and nerve diseases in diabetic and non-diabetic patients using Metabolic Activation Therapy improving or arresting the symptoms of peripheral neuropathy, autonomic neuropathy and eye disease. In all of the above detailed patents, the respiratory quotient (RQ) of the patient is used to monitor/measure therapeutic efficacy and provides physiological evidence that the treatment is effective. WO 03/084459 details a way the measurement of circulating glucose can be used as an alternative measure of therapeutic efficacy providing physiological evidence that the treatment is effective. Additionally, WO 03/084459 introduces the use of Metabolic Activation Therapy to improve one or more of confusion, weakness, disorientation, or cognitive function in diabetic patients suffering with senile dementia. Metabolic Activation Therapy is well known by those skilled in the art for improving energy levels in treated subjects.

US 2006/0122099 details a method for improving total body tissue glucose processing in subjects wherein the method is used for lowering levels of hemoglobin A1c, delaying the onset or slowing the progression of diabetes related nephropathy, retinopathy, neuropathy, cardiovascular disease, heart disease, treating wounds, promoting healing and avoiding amputations, improving mental function in subjects with senile dementia or having a decreased glucose oxidation rate due to aging, brain injury or jet lag.

Other stress inducing non-surgical medical procedures include but are not limited to: lung biopsy; multiple stent and/or angioplasty placement; administration of chemotherapy or intravenous medications such as steroids and other hormones; medications antagonizing the action of insulin such as glucocorticoids; medications suppressing secretion of insulin including potassium-wasting diuretics such as thiazide (hydrochlorothiazide) and loop diuretics (e.g. furosemide) as well as phenytoin (Dilantin); and medications with miscellaneous effects on hyperglycemia including azathioprine, calcium channel blockers, chemotherapeutic medications, chlorpromazine, diazoxide (Hyperstat), olanzapine (Zyprexa), and propranolol.

Accordingly, the present invention is an insulin dosage regimen for administering insulin to diabetic and nondiabetic patients by infusing a series of intravenous pulses of insulin into them at regular intervals. At the same time the patient ingests a carbohydrate containing meal or other meal capable of increasing circulating glucose levels in the patient and circulating glucose measurements are made periodically to insure the patient does not become hypoglycemic and also that hepatic and other body tissues processing of glucose have been restored.

Liquid or food containing glucose is consumed by the patient to provide the second signal to activate the liver and to prevent the patient from becoming hypoglycemic. The preferred liquid or food containing glucose is 2 to 10 ounces of GLUCOLA which translates to 20 to 100 grams of glucose, but any similar type of liquid or high glycemic food, including but not limited to cake and bread, containing glucose may be given to the patient. For non-diabetic patients ingested amounts may be higher and should be adjusted for each patient.

In the preferred embodiment of the invention, a programmable insulin pump is programmed to deliver intravenous insulin in precisely measured pulses, programmed to deliver each of those pulses within a minimum amount of time, and to allow for timed intervals between each pulse. However, any method of infusing measured amounts of insulin to include simple injection with a syringe is also acceptable. The preferred means of insulin delivery is a programmable infusion device capable of providing measured pulses of insulin on a prearranged interval, so long as there is sufficient glucose in the blood to keep the patient from becoming hypoglycemic. It is also preferable that the infusion device is capable of delivering the pulses of insulin in as short duration of time as possible, without adversely affecting the vein at the site of infusion used. Any infusion device including a simple syringe may be used to deliver the pulses and achieve the needed infusion profile.

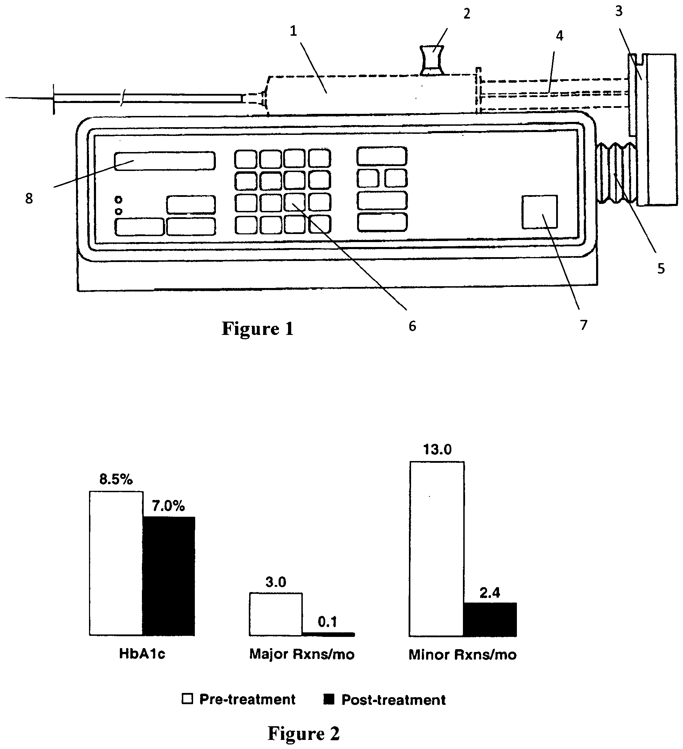

In one embodiment a programmable, handheld syringe pump uses a conventional medical syringe for infusing the insulin. Referring to **FIG. 1** the syringe 1 is attached to the pump by the syringe holder 2 and the blocking plate 3. The plunger 4 is activated by the syringe driver 5. The syringe driver is actuator driven by any of a number of possible actuator configurations known to those skilled in the art. Any conventional infusion tube connection device may be used to connect the infusion tube to the syringe. Programmed values can be inputted to a control processor via the keyboard 6, through firmware in the pump or by software via a communications link 7 to a higher level computer or any other appropriate input method. Status, alarm and other messages can be read on the pump's display 8. Circulating glucose measurements are made periodically. Typical circulating glucose sensors include but are not limited to finger stick devices, non-invasive instruments using near infrared spectroscopy or radio frequency, and intravenous and implanted sensors. A communications link 7 between the circulating glucose meter and the infusion device providing current circulating glucose levels can be used to determine when therapeutic efficacy has been obtained or to sound an alarm when circulating glucose levels become dangerously high or low. Any other method for either directly or indirectly obtaining an accurate measure of the change in circulating glucose levels is also acceptable. The communications link 7 may be used to send alarm, diagnostic and status messages to a higher level computer via any acceptable communications protocol and medium as well.

When the pump is activated, it dispenses the programmed pulse of insulin in the programmed amount of time to the subject. The insulin travels through the infusion tube, to the needle, which is inserted intravenously into the subject wherever convenient but preferably in the forearm. The time to deliver each pulse should be as short as possible and at least less than one minute and preferably on the order of seconds. Pump status, alarm status and circulating-glucose levels, among other parameters of the system may be displayed on the display panel 8.

Although a syringe type pump is used as an example of a typical way to deliver insulin to the patient, there are any of a number of infusion pumps available in the market and well-known by those skilled in the art that can be used alternatively.

In the preferred embodiment, insulin pulses are delivered to a patient utilizing Metabolic Activation Therapy as follows: On the morning of the procedure, the patient is preferably seated in a blood drawing chair and a 23 gauge needle or catheter is preferably inserted into a hand or forearm vein to obtain vascular access. However, any system of such access may accomplish the needed result, including but not limited to indwelling catheters, PICC lines and PortaCaths. After a short equilibration period, the patient is asked to make a circulating glucose measurement prior to starting the actual infusion of insulin. It is preferable that patients have circulating glucose levels close to 200 mg/dl prior to using the infusion device. In the case of pregnant diabetic women, however, every attempt is made to keep the maximum circulating glucose level to 180 mg/dl or less.

After the circulating glucose measurement has been taken and the patient has the proper circulating glucose starting level, the patient is asked to consume a liquid or food containing glucose. The amount of glucose given to the diabetic patient ranges from 20 to 100 grams or 2 to 10 ounces of Glucola. Any food which will increase the circulating glucose levels of the subject may also be used. However, the amount of initial glucose given to the patient may vary. In the non-diabetic patient more glucose may be required than in the diabetic patient, but the other parameters would remain the same, including the need for a pulsed delivery. Pulses of insulin are then administered intravenously at planned intervals of time by any of a number of pulsed insulin delivery pumps well known by those skilled in the art, usually every six minutes; however, other intervals may be used from as low as every three minutes up to every 30 minutes. A series of pulses will be completely delivered over a period of about a minimum of 6 minutes and a maximum of about 180 minutes. For diabetic patients the amount of insulin in each pulse is 5-200 milliunits of insulin per kilogram of body weight; for non-diabetic patients the amount of insulin may be slightly lower on the order of 1 to 200 milliunits of insulin per kilogram of body weight.

In one preferred embodiment RQ measurements using a Sensormedic Metabolic Measurement Cart or equivalent RQ instrument are obtained before and throughout the treatment period at 60-minute intervals. An increase in the RQ to greater than 0.88 is used as the index of therapeutic efficacy to insure that activation has occurred. Because RQ measurements are time consuming and expensive, measurements of RQ are conducted before insulin is infused and shortly after the start of the daily Metabolic Activation Therapy to insure that the patient is responding properly. Thereafter circulating plasma glucose measurements may replace RQ measurements at the discretion of the attending physician.

Circulating plasma glucose measurements are made as frequently as possible. When finger stick measurements are used, because of the discomfort to the patient, it is recommended that readings be taken every 30 minutes or more frequently. At the discretion of the attending physician when circumstances dictate, readings can be taken at intervals longer than 30 minutes. When less invasive methods of measuring circulating glucose are used, readings can be taken more frequently, preferably after the infusion of each pulse of insulin. It is recommended that a period of one to two minutes is allowed after the infusion of each pulse of insulin before circulating glucose levels are measured. The circulating glucose level will typically rise by approximately 100 to 150 mg/dl at the beginning of the insulin infusion due to the concomitant ingestion of glucose, before starting to fall.

In patients whose hepatic glucose processing has been restored, there will be a fall in circulating glucose levels by about 50-100 mg/dl. Hence a falling glucose level of 50 mg/dl can be used as an alternative index of therapeutic efficacy to the RQ measurement. In patients who have yet to obtain proper hepatic glucose processing, there will be no fall or a fall considerably less than 50 mg/dl. The fall in circulating glucose levels, indicating restoration of hepatic processing of glucose, is generally achieved within one hour of initiation of the first pulse of insulin using the preferred embodiment of this invention; however, the time required may be shorter or longer than one hour. It is possible to decrease the amount of insulin in each pulse and to lengthen the time between pulses to adjust the amount of time required for a drop of 50 mg/dl to occur. In one preferred embodiment of the current invention the maximum time for the circulating glucose levels to fall 50 mg/dl occurs in less than 2 hours after the delivery of the first pulse of the series of pulses. This decrease in circulating glucose is caused by the combination of increased glucose utilization by muscle and the uptake and use of glucose by the liver.

The phase during which a series of pulses of insulin is administered and glucose ingested lasts typically for 56 minutes (ten pulses with a six-minute interval between pulses) and is followed by a rest period of usually 1/2 to two hours. The rest period allows the elevated insulin levels to return to baseline. During periods when insulin is not being infused, the intravenous site is preferably converted to a heparin or saline lock. The entire procedure is repeated until the desired effect is obtained. Typically the procedure is repeated three times for each treatment day, but can be repeated as few as two times and up to 8 times in one day. Prior to the patient being discharged from the procedure, in the preferred embodiment circulating glucose levels stabilize at 100-200 mg/dl for approximately 30-45 minutes.

Hepatic processing of glucose includes proper uptake of glucose by the liver cells, oxidation of glucose by the liver cells, storage of glucose as hepatic glycogen in the liver cells, and conversion of glucose to fat or alanine, an amino acid, by the liver cells. Hepatic processing is impaired when the liver fails to produce certain hepatic enzymes, including but not limited to hepatic glucokinase, phosphofructokinase, and pyruvate kinase or to activate other hepatic enzymes including but not limited to pyruvate dehydrogenase needed for proper glucose processing. Impaired processing of glucose is a fundamental condition of type 1 and type 2 diabetic patients, for non-diabetic patients whose pancreas is not producing sufficient insulin, and for non-diabetic patients experiencing significant insulin resistance, or a combination of these factors.

Metabolic Activation Therapy when used for long periods of time with "brittle" patients showed an amazing outcome. Example 1 below describes that outcome.

### Example 1: Decreased HbA1c Readings Accompanied by a Major Decrease in Major Hypoglycemic Events in "Brittle" Diabetic Patients

In a study published in 1993 [Aoki TT, Benbarka MM, Okimura MC, Arcangeli MA, Walter RM Jr., Wilson LD, Truong M, Barber AR, Kumagai LF: "Long-term Intermittent Intravenous Insulin Therapy and Type 1 Diabetes Mellitus." Lancet 1993; 342: 515-518.], the results of long term Metabolic Activation Therapy on 20 IDDM patients with "brittle" disease (wide swings in plasma glucose concentrations and frequent hypoglycemic episodes despite being on intensive subcutaneous insulin therapy- four daily insulin injection regimen-for at least 1 year) were reported. The patients received an average of 41 months of Metabolic Activation Therapy. **FIG. 2** shows a graphical representation of the results of the study. The major results of the study indicated:
a. A significant decrease in HbA1c from the baseline of 8.5% to 7.0% at the end of the observation period (p<0.0003)
b. A decline in the frequency of major hypoglycemic events from 3.0 to 0.1/ month P<.0001).
c. A decline in the frequency of minor hypoglycemic events from 13.0 to 2.4/ month (p<0.0001)

The last two effects are contrary to the results of the Diabetes Control and Complication Trial (DCCT) where the improvement in HbA1c was accompanied by a threefold increase in the frequency of major hypoglycemic events. and points to a significant barrier in the attempt to control blood glucose levels without the additional threat of hypoglycemic reactions. In contrast, the above data show that tight glucose control is not necessarily accompanied by an increase in hypoglycemia. In fact, the numbers of major and minor hypoglycemic events were significantly decreased.

Although the treatment is typically used to treat patients on a long term basis to successfully treat all complications of diabetes, it was thought that using Metabolic Activation Therapy for as short a period as one to two weeks prior to an impending surgery and other stress inducing procedures including those requiring hospitalization might help to mitigate the problems of hypoglycemia as a negative side effect of using IV insulin and glucose to treat patients undergoing surgery and other stress inducing procedures including those requiring hospitalization. Example 2 below describes the results of treating 10 patients with Metabolic Activation Therapy and comparing those patients with 10 control patients not receiving such treatments.

### Example 2: Using Metabolic Activation Therapy Preoperatively in Type 2 Diabetic Patients Scheduled for Advance Colorectal Cancer Operations

In this study, the metabolic impact of preemptive inpatient preoperative Metabolic Activation Therapy on postoperative glycemic control and insulin requirements in elderly type 2 diabetic patients undergoing colorectal resection for advanced colorectal cancer was studied. It was postulated that MAT, if successful in decreasing insulin resistance and/or increasing insulin action postoperatively, should result in improved glucose control with less administered insulin.

Twenty elderly, well-controlled type 2 diabetic patients scheduled for colorectal resections were randomly assigned to groups that received 1) MAT treatment 2 successive days 1 week prior to surgery or 2) did not receive (control group) preoperative inpatient MAT treatment. Plasma glucose (PG) levels were measured premeal and at bedtime before, and daily for 7 days after surgery. Insulin requirements per 1 g infused glucose (I/G ratio) and per given total carbohydrate ingested and infused (I/C ratio) were evaluated.

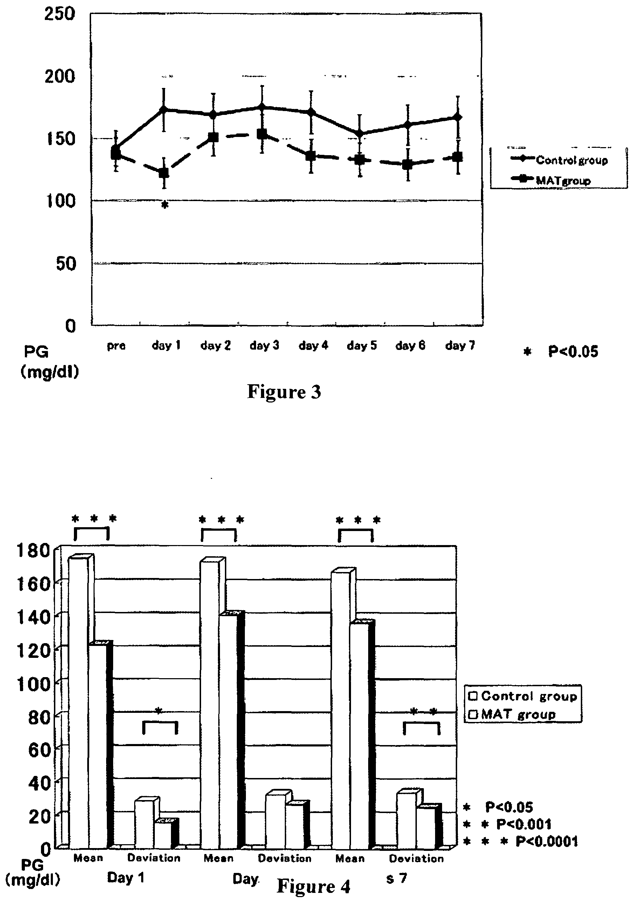

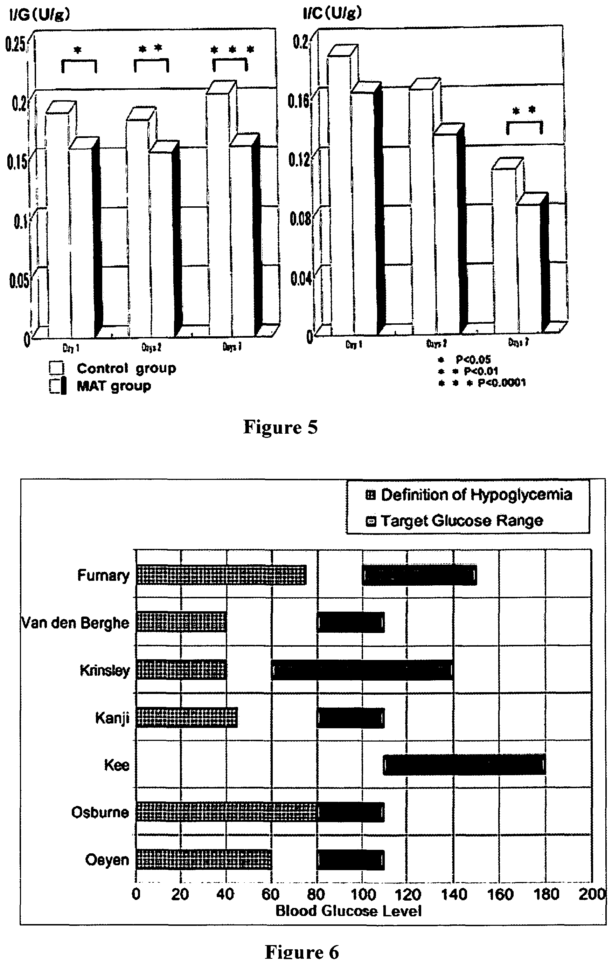

Mean PG levels before and during the study **(****FIG. 3****)** and on postoperative day 1 (123±33 versus 175±56 mg/dL; P < 0.0001), during the first 2 postoperative days (141±42 versus 173±54 mg/dL; P < 0.0001), and during the entire 7 day period (136±38 versus 167±52 mg/dL; P < 0.0001) **(****FIG. 4****)** were significantly lower in MAT patients than in controls. Hypoglycemia frequency did not differ significantly between the groups. Postoperative mean I/G and I/C ratios **(****FIG. 5****)** were significantly lower in MAT patients than in control patients over the entire 7 day period.

Since preoperative MAT significantly lowered postoperative plasma glucose levels with significantly less insulin, it was concluded that MAT administered preoperatively uniquely decreased insulin resistance and improved insulin action in the postoperative state. These results suggest that wound healing should be accelerated in the MAT treated group thereby shortening hospital stays.

When examining patient safety issues regarding insulin infusions and tight glycemic control during and after surgery, different published studies set different definitions of what the target glucose range should be and when a patient is determined to be hypoglycemic. **FIG. 6** demonstrates how much difference there is in implementing IV insulin during surgical procedures. Also, in most of the studies, there was a gap between the lower level of target plasma glucose and the upper range of hypoglycemia. Some studies defined this area as low, whereas others left it undefined. When a study patient had a plasma glucose result that fell into this area, the patient may have been experiencing clinical signs of hypoglycemia that are not reported as a hypoglycemic event according to the protocol.

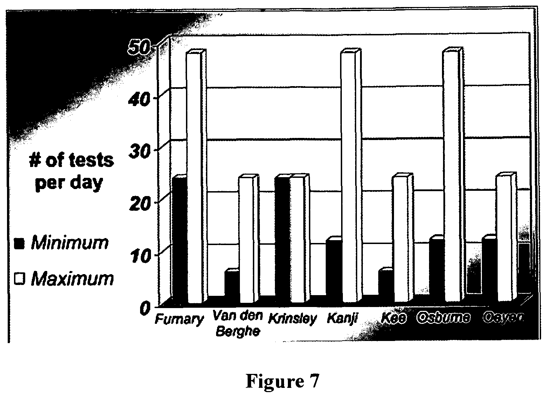

FIG. 7 demonstrates for each of the published studies shown in **FIG. 6** the number of times plasma glucose must be monitored by nurses attending patients. There are a high number of plasma glucose measurements required every day, varying from 6, or once every four hours, to 48 or once every 30 minutes. The responsibility for testing the patient and then changing the regimen of insulin and glucose infusion is left to attending nurses, is time consuming and subject to considerable error. However, it is universal that attempting to control hyperglycemia requires considerable effort and costs and there are inevitable mistakes or failures to make timely measurements and changes to the treatment regimen.

In cardiovascular surgery, surgical intensive care patient populations, and colorectal resection in patients with diabetes mellitus, improved glucose control during the 48 h immediately following surgery has been clearly linked with a reduction in the rate of major infections. This association appears to be independent of preoperative glycemic control and the dose of insulin infused postoperatively. Thus, surgery in diabetic patients is complicated by the need for metabolic control, especially during the immediate post-surgical period.

Although many intravenous insulin infusion protocols have been developed for the management of diabetic patients during surgery and other stress inducing procedures including those requiring hospitalization and in critical care units, there is no consensus on the best way to achieve optimal results in terms of glycemic control without important side effects, including severe hypoglycemia.

Secondary causes of hyperglycemia result from non-diabetic insulin resistance, toxins including medications including steroids, severe pain, infections, pancreatic diseases of various kinds, and endocrine dysfunction. Insulin resistance may occur due to medications, age, ethnicity, obesity, lack of exercise, cirrhosis of the liver, and/or stress. Toxins that can cause elevated blood glucose levels include but are not limited to alcohol, prescription medications, and the metal cadmium. Certain medications increase the risk of hyperglycemia, including beta blockers, epinephrine, thiazide diuretics, corticosteroids, niacin, pentamidine, protease inhibitors, L-asparaginase, and some antipsychotic agents. The acute administration of stimulants such as amphetamine typically produces hyperglycemia. Some of the newer, double action anti-depressants such as Zyprexa, and Cymbalta, can also cause significant hyperglycemia.

Diseases of the exocrine pancreas that can cause high plasma glucose include a hereditary condition called hemochromatosis as well as pancreatic cancer. Hemochromatosis causes iron excess in the body. This then causes hyperglycemia due to the toxicity of iron to insulin-producing pancreatic beta cells. Hemochromatosis is often asymptomatic when detected on routine examinations. If the disease has progressed to the point of causing hyperglycemia, other symptoms may be expected including fatigue, cirrhosis, skin pigmentation, cardiomyopathy, arthropathy (usually of the hands) and/or hypogonadism.

Pancreatic islet cell tumors may cause hypoglycemia. Commonly presenting symptoms of pancreatic cancer include jaundice, mid-abdominal pain radiating to the back, steatorrhea (fatty diarrhea), and anorexia (loss of appetite) and weight loss.

Several forms of endocrine dysfunction may cause hyerglycemia, including acromegaly, hyperthyroidism, hypercortisolism and pheochromocytomas. Excess growth hormone can cause a condition called acromegaly. Excess levels of growth hormone cause hyperglycemia, enlargement of peripheral body parts, arthralgias, and even gout.

Glucose toxicity is a phenomenon which can contribute to high plasma glucose in some individuals. Glucose toxicity is the condition in which initial hyperglycemia, resulting from any cause, may itself cause further high glucose levels by decreasing insulin sensitivity and increasing glucose production in the liver.

A high proportion of patients suffering an acute metabolic/biochemical stress such as stroke or myocardial infarction may develop hyperglycemia even in the absence of a diagnosis of diabetes. Human and animal studies suggest that this is not benign, and that stress-induced hyperglycemia is associated with a high risk of mortality after both stroke, acute coronary syndrome, and myocardial infarction Plasma glucose >120 mg/dl in the absence of diabetes may be a clinical sign of sepsis.

Physical trauma, surgery and other stress inducing procedures including those requiring hospitalization and many forms of severe stress can temporarily increase glucose levels.

Hyperglycemia frequently accompanies infection and inflammation. When the body is stressed, endogenous catecholamines, amongst other things, are released to raise the plasma glucose levels. The amount of increase varies from person to person and from inflammatory response to response.

The insulin treatment regimen of the current invention can be used to biochemically prepare diabetic and non-diabetic patients for surgery and other stress inducing procedures including those requiring hospitalization, while minimizing both hyperglycemia and hypoglycemia during and after the surgery or other stress inducing procedures including those requiring hospitalization. The insulin treatment regimen allows for restoration toward normality of the biochemistry and physiology of these individuals resulting in the control of plasma glucose to levels lower than can currently be obtained with minimal threat of hypoglycemia. The stress on nursing personnel to make up to 48 or more accurate plasma glucose measurements a day, calculate appropriate insulin doses and glucose amounts to be administered, and then administer both could result in a considerable number of mistakes and subsequent harm to the patient. The preoperative insulin treatment regimen prepares the patients biochemically/physiologically for a given surgical procedure or other stress inducing procedures including those requiring hospitalization thereby obviating the necessity for achieving "tight" glucose control during and after surgery and other stress inducing procedures including those requiring hospitalization. As a result of such preemptive treatment, there should be fewer nursing errors and less potentially damaging outcomes to the patients. Finally, the insulin treatment regimen lowers overall medical costs by decreasing hospital length of stay.

## Claims

1. Insulin for use in a method of treatment of a subject to prepare the subject for a scheduled surgery or other stress inducing procedures including those requiring hospitalization which subject is experiencing elevated circulating glucose levels or suffers from diabetes, said method being a preoperative Metabolic Activation Therapy comprising the steps of:
ingestion by the subject of a carbohydrate containing meal capable of increasing circulating glucose levels in said subject;
periodic monitoring of circulating glucose levels;
delivery to the subject of a series of intravenous pulses of insulin by an infusion device; wherein an amount of said insulin in each pulse, an interval between pulses and an-amount of time to deliver each pulse to the subject are selected so that a monitoring device to measure therapeutic efficacy provides physiological evidence that the treatment is effective and **characterized in that** the preoperative non long term insulin treatment regimen prepares the patient biochemically/physiologically for a given surgical procedure or other stress inducing procedure including those requiring hospitalization to uniquely decrease insulin resistance and improve insulin action in the postoperative state thereby obviating the necessity for achieving tight glucose control during and after surgery or other stress inducing procedures including those requiring hospitalization.

2. Insulin according to claim 1, wherein the monitoring device to measure therapeutic efficacy indicates that the subject's circulating glucose level initially rises and then falls by an amount equal to 50 mg/dl or more.

3. Insulin according to claim 1, wherein the monitoring device to measure therapeutic efficacy indicates that the subject's respiratory quotient (RQ) rises to a level higher than 0.88.

4. Insulin according to any preceding claim, wherein the said delivery of a series of pulses is performed 2 to 8 times per treatment day.

5. Insulin according to claim 4, wherein the subject is treated for one or more treatment days prior to surgery or other stress inducing procedures including those requiring hospitalization.

6. Insulin according to any preceding claim wherein said infusion device comprises a syringe to deliver pulses of insulin.

7. Insulin according to claim 2 wherein the falling of the circulating glucose level in the subject by an amount equal to 50mg/dl or more occurs within two hours of administering the first pulse of the series of pulses.

8. Insulin according to any preceding claim wherein the amount of insulin in each pulse is between 1 and 200 milliunits per kilogram of body weight.

9. Insulin according to any of claims 1 to 7, wherein the patient suffers from diabetes and the amount of insulin in each pulse is between 5 and 200 milliunits per kilogram of body weight.

10. Insulin according to any preceding claim, wherein said infusion device comprises a programmable processor unit and the amount of insulin in each pulse, the time interval between pulses and the amount of time to deliver each pulse to the subject are controlled by said programmable processor unit.

11. Insulin according to any preceding claim, wherein one said series of pulses is delivered over a period of 6 to 180 minutes.

12. Insulin according to any preceding claim, wherein each pulse of the said series of pulses is delivered every 3 to 30 minutes.

13. Insulin according to any preceding claim, wherein said carbohydrate containing meals contains between 20 to 100 grams of glucose.

14. Insulin according to any preceding claim wherein information is transferred automatically between a circulating glucose meter and said infusion device.

## Patentansprüche

1. Insulin für die Verwendung in einem Behandlungsverfahren für einen Patienten, um den Patienten auf eine geplante Operation oder andere stressinduzierende Maßnahmen einschließlich solcher Maßnahmen, die einen Krankenhausaufenthalt erfordern, vorzubereiten, wobei der Patient erhöhte zirkulierende Glukosewerte aufweist oder an Diabetes leidet, wobei das Verfahren eine präoperative Stoffwechselaktivierungsbehandlung ist, die folgende Schritte umfasst:
Einnehmen einer kohlenhydrathaltigen Mahlzeit durch den Patienten, die fähig ist, die zirkulierenden Glukosewerte des Patienten zu erhöhen;
regelmäßiges Überwachen der zirkulierenden Glukosewerte;
Verabreichen einer Reihe von intravenösen Insulinimpulsen an den Patienten mithilfe einer Infusionsvorrichtung;
wobei eine Menge an Insulin in jedem Impuls, ein Zeitabstand zwischen den Impulsen und ein Tempo, in dem jeder Impuls dem Patienten verabreicht wird, so ausgewählt werden, dass eine Überwachungsvorrichtung zur Messung der therapeutischen Wirksamkeit den physiologischen Beweis bereitstellt, dass die Behandlung wirksam ist, und **dadurch gekennzeichnet, dass** die präoperative nicht-langfristige Insulinbehandlungsmethode den Patienten biochemisch/physiologisch auf einen bestimmten chirurgischen Eingriff oder andere stressinduzierende Maßnahmen einschließlich solcher Maßnahmen, die einen Krankenausaufenthalt erfordern, vorbereitet, um die Insulinresistenz auf einzigartige Weise zu verringern und die Insulinwirkung im postoperativen Zustand zu verbessern und dadurch die Notwendigkeit einer strengen Glukosekontrolle während und nach der Operation oder anderen stressinduzierenden Maßnahmen einschließlich solcher Maßnahmen, die einen Krankenhausaufenthalt erfordern, zu umgehen.

2. Insulin nach Anspruch 1, wobei die Überwachungsvorrichtung zur Messung der therapeutischen Wirksamkeit anzeigt, dass die zirkulierenden Glukosewerte des Patienten anfänglich steigen und dann um eine Menge von 50 mg/dl oder mehr fallen.

3. Insulin nach Anspruch 1, wobei die Überwachungsvorrichtung zur Messung der therapeutischen Wirksamkeit anzeigt, dass der respiratorische Quotient (RQ) des Patienten auf einen Wert von mehr als 0,88 ansteigt.

4. Insulin nach einem der vorhergehenden Ansprüche, wobei das Verabreichen einer Reihe von Impulsen 2 bis 8 mal pro Behandlungstag ausgeführt wird.

5. Insulin nach Anspruch 4, wobei die Behandlung des Patienten einen oder mehrere Behandlungstage vor der Operation oder anderen stressinduzierenden Maßnahmen einschließlich solcher Maßnahmen, die einen Krankenausaufenthalt erfordern, beginnt.

6. Insulin nach einem der vorhergehenden Ansprüche, wobei die Infusionsvorrichtung eine Spritze zur Verabreichung der Insulinimpulse umfasst.

7. Insulin nach Anspruch 2, wobei das Fallen der zirkulierenden Glukosewerte des Patienten um eine Menge von 50 mg/dl oder mehr innerhalb von zwei Stunden nach Verabreichen des ersten Impulses der Reihe von Impulsen auftritt.

8. Insulin nach einem der vorhergehenden Ansprüche, wobei die Menge an Insulin in jedem Impuls sich zwischen 1 und 200 Millieinheiten pro Kilogramm Körpergewicht bewegt.

9. Insulin nach einem der Ansprüche 1 bis 7, wobei der Patient an Diabetes leidet und die Menge an Insulin in jedem Impuls sich zwischen 5 und 200 Millieinheiten pro Kilogramm Körpergewicht bewegt.

10. Insulin nach einem der vorhergehenden Ansprüche, wobei die Infusionsvorrichtung eine programmierbare Verarbeitungseinheit umfasst und die Menge an Insulin in jedem Impuls, der Zeitabstand zwischen den Impulsen und das Tempo, mit dem jeder Impuls dem Patienten verabreicht wird, von der programmierbaren Verarbeitungseinheit gesteuert wird.

11. Insulin nach einem der vorhergehenden Ansprüche, wobei eine dieser Reihen von Impulsen über einen Zeitraum von 6 bis 180 Minuten verabreicht wird.

12. Insulin nach einem der vorhergehenden Ansprüche, wobei jeder Impuls der Reihe von Impulsen alle 3 bis 30 Minuten verabreicht wird.

13. Insulin nach einem der vorhergehenden Ansprüche, wobei die kohlenhydrathaltigen Mahlzeiten zwischen 20 und 100 Gramm Glukose enthalten.

14. Insulin nach einem der vorhergehenden Ansprüche, wobei Informationen automatisch zwischen einem Messgerät der zirkulierenden Glukosewerte und der Infusionsvorrichtung übertragen werden.

## Revendications

1. Insuline à utiliser dans une méthode de traitement d'un sujet pour préparer le sujet à une intervention chirurgicale programmée ou à d'autres procédures faisant intervenir un stress comprenant des procédures exigeant une hospitalisation, ledit sujet subissant des niveaux élevés de glucose circulant ou souffrant de diabètes, ladite méthode consistant en une thérapie préopératoire d'activation métabolique, comprenant les étapes consistant à :
ingérer, par le sujet, un repas contenant des carbohydrates permettant d'augmenter des niveaux de glucose circulant chez ledit sujet ;
surveiller périodiquement les niveaux de circulation de glucose ;
administrer au sujet une série de pulsations intraveineuses d'insuline au moyen d'un dispositif d'infusion ;
dans laquelle on sélectionne une quantité de ladite insuline de chaque pulsation, un intervalle entre des pulsations et une durée d'administration de chaque pulsation au sujet de sorte qu'un dispositif de surveillance servant à mesurer une efficacité thérapeutique fournisse une preuve physiologique de l'efficacité du traitement, et **caractérisée en ce que** le régime à court terme de traitement préopératoire à l'insuline prépare biochimiquement /physiologiquement le patient à une procédure chirurgicale donnée ou à une autre procédure faisant intervenir un stress comprenant des procédures exigeant une hospitalisation de façon à diminuer uniquement la résistance à l'insuline et à améliorer l'action de l'insuline dans l'état post-opératoire, évitant ainsi la nécessité d'effectuer un contrôle strict de glucose pendant et après l'intervention chirurgicale ou d'autres procédures faisant intervenir un stress comprenant des procédures exigeant une hospitalisation.

2. Insuline selon la revendication 1, dans laquelle le dispositif de surveillance servant à mesurer l'efficacité thérapeutique indique que le niveau de glucose circulant du sujet monte initialement et baisse par la suite d'une valeur supérieure ou égale à 50 mg/dl.

3. Insuline selon la revendication 1, dans laquelle le dispositif de surveillance servant à mesurer l'efficacité thérapeutique indique que le quotient respiratoire métabolique du sujet (RQ) monte à un niveau supérieur à 0,88.

4. Insuline selon l'une quelconque des revendications précédentes, dans laquelle ladite administration d'une série de pulsations est effectuée 2 à 8 fois par jour de traitement.

5. Insuline selon la revendication 4, dans laquelle le sujet et traité pendant un ou plusieurs jours de traitement avant une intervention chirurgicale ou d'autres procédures faisant intervenir un stress comprenant des procédures exigeant une hospitalisation.

6. Insuline selon l'une quelconque des revendications précédentes, dans laquelle ledit dispositif d'infusion comprend une seringue servant à administrer des pulsations d'insuline.

7. Insuline selon la revendication 2, dans laquelle la baisse du niveau de glucose circulant du sujet, d'une valeur supérieure ou égale à 50 mg/dl, se produit dans les deux heures qui suivent l'administration de la première pulsation de la série de pulsations.

8. Insuline selon l'une quelconque des revendications précédentes, dans laquelle la quantité d'insuline de chaque pulsation est comprise entre 1 et 200 milli-unités par kilogramme de poids corporel.

9. Insuline selon l'une quelconque des revendications 1 à 7, dans laquelle le patient souffre de diabètes, et la quantité d'insuline de chaque pulsation est comprise entre 5 et 200 milli-unités par kilogramme de poids corporel.

10. Insuline selon l'une quelconque des revendications précédentes, dans laquelle ledit dispositif d'infusion comprend une unité processeur programmable, et ladite unité processeur programmable commande la quantité d'insuline de chaque pulsation, l'intervalle de temps entre des pulsations et la durée d'administration de chaque pulsation au sujet.

11. Insuline selon l'une quelconque des revendications précédentes, dans laquelle une série de pulsations de ladite série de pulsations est administrée sur une période de 6 à 180 minutes.

12. Insuline selon l'une quelconque des revendications précédentes, dans laquelle chaque pulsation de ladite série de pulsations est délivrée toutes les 3 à 30 minutes.

13. Insuline selon l'une quelconque des revendications précédentes, dans laquelle lesdits repas contenant des carbohydrates contiennent entre 20 et 100 grammes de glucose.

14. Insuline selon l'une quelconque des revendications précédentes, dans laquelle des informations sont transférées automatiquement entre un dispositif de mesure de glucose circulant et ledit dispositif d'infusion.
